(19) Europäisches Patentamt
European Patent Office
Office européen des brevets

(11) **EP 3 636 271 B1**

(12) **EUROPEAN PATENT SPECIFICATION**

(45) Date of publication and mention
of the grant of the patent:
**19.11.2025 Bulletin 2025/47**

(21) Application number: **18813352.4**

(22) Date of filing: **08.05.2018**

(51) International Patent Classification (IPC):
*A61K 35/28* (2015.01)          *A61K 35/35* (2015.01)
*A61K 38/17* (2006.01)          *A61K 35/51* (2015.01)
*A61P 9/04* (2006.01)

(52) Cooperative Patent Classification (CPC):
**A61K 35/28; A61K 35/35; A61K 35/51;
A61K 38/17; A61P 9/04**

(86) International application number:
**PCT/JP2018/017807**

(87) International publication number:
**WO 2018/225440 (13.12.2018 Gazette 2018/50)**

(54) **THERAPEUTIC AGENT FOR DILATED CARDIOMYOPATHY**

THERAPEUTIKUM FÜR DILATIERTE KARDIOMYOPATHIE

AGENT THÉRAPEUTIQUE CONTRE LA CARDIOMYOPATHIE DILATÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.06.2017 JP 2017114240**

(43) Date of publication of application:
**15.04.2020 Bulletin 2020/16**

(73) Proprietor: **Rohto Pharmaceutical Co., Ltd.
Osaka-shi
Osaka 544-8666 (JP)**

(72) Inventors:
• **SAWA Yoshiki**
**Suita-shi**
**Osaka 565-0871 (JP)**
• **MIYAGAWA Shigeru**
**Suita-shi**
**Osaka 565-0871 (JP)**
• **KAJITA Daisuke**
**Suita-shi**
**Osaka 565-0871 (JP)**
• **KURATA Hayato**
**Osaka-shi**
**Osaka 544-8666 (JP)**
• **TAMADA Kotoe**
**Osaka-shi**
**Osaka 544-8666 (JP)**
• **NISHIDA Hiroyuki**
**Osaka-shi**
**Osaka 544-8666 (JP)**

(74) Representative: **Mewburn Ellis LLP
Aurora Building
Counterslip
Bristol BS1 6BX (GB)**

(56) References cited:
WO-A1-2014/013029          WO-A1-2016/094932
WO-A2-2015/076717          JP-A- 2005 035 945
US-A1- 2011 003 008          US-A1- 2015 125 950

• N. NAGAYA: "Transplantation of Mesenchymal
Stem Cells Improves Cardiac Function in a Rat
Model of Dilated Cardiomyopathy",
CIRCULATION, vol. 112, no. 8, 23 August 2005
(2005-08-23), US, pages 1128 - 1135,
XP055270979, ISSN: 0009-7322, DOI: 10.1161/
CIRCULATIONAHA.104.500447

- ANONYMOUS: "Mesenchymal Stem Cells for Idiopathic Dilated Cardiomyopathy", 8 October 2013 (2013-10-08), XP055767063, Retrieved from the Internet <URL:https://clinicaltrials.gov/ct2/show/NCT01957826?term=mesenchymal+stem+cells&cond=Dilated+Cardiomyopathy&draw=2&rank=3> [retrieved on 20210120]
- SAHOO, S. ET AL.: "Exosomes and cardiac repair after myocardial infarction", CIRC RES., vol. 114, no. 2, 2014, pages 333 - 344, XP055554390, ISSN: 0009-7330
- ZHANG, H. ET AL.: "Inhibition of myocardial ischemia/reperfusion injury by exosomes secreted from mesenchymal stem cells", STEM CELL INT., vol. 2016, 2016, pages 1 - 8, XP055554411, ISSN: 1687-966X
- LAI, RC. ET AL.: "Exosome secreted by MSC reduces myocardial ischemia/reperfusion injury", STEM CELL RES., vol. 4, 2010, pages 214 - 222, XP027054731, ISSN: 1873-5061
- CHANG, YL. ET AL.: "Therapeutic potential of stem cells strategy for cardiovascular diseases", STEM CELL INT., vol. 2016, 2016, pages 1 - 10, XP055554414, ISSN: 1687-966X
- LIANG, H. ET AL.: "Increased expression of pigment epithelium-derived factor in aged mesenchymal stem cells impairs their therapeutic efficacy for attenuating myocardial infarction injury", EUR HEART J., vol. 34, no. 22, 2 June 2013 (2013-06-02), pages 1681 - 1690, XP055082889, ISSN: 0195-668X

Remarks:

The file contains technical information submitted after the application was filed and not included in this specification

**Description**

TECHNICAL FIELD

The present invention relates to an agent for use in treating dilated cardiomyopathy.

BACKGROUND ART

Dilated cardiomyopathy (DCM) is a group of diseases of idiopathic cardiomyopathy, which is characterized by myocardial contractile dysfunction and dilation of the left ventricular lumen, and is progressive in most cases. Dilated cardiomyopathy is also characterized by a chronic heart failure symptom, and is often associated with a poor prognosis and recurrent acute exacerbation. Furthermore, the disease may lead to sudden death due to fatal arrhythmia, or arterial thromboembolism. Dilated cardiomyopathy is a disease group in which inheritable ones and acquired ones are mixed. By definition, the disease is morphologically and functionally classified, and clinically there are many similar diseases in which "dilation of the left ventricle" and "left ventricular systolic dysfunction" occur. Therefore, it is necessary to exclude those similar other specific cardiomyopathies when investigating dilated cardiomyopathy.

Although the etiology of dilated cardiomyopathy has long been unknown, involvement of virus infection and autoimmune abnormality, as well as hereditary predisposition, have been identified, and various types of abnormal cell-mediated immunity have been observed. It is reported that the number of regulatory T cells is decreased and instead the number of helper T cells is increased in serum of a patient with dilated cardiomyopathy. A study using a myocardial biopsy specimen shows that MHC class II molecules have been expressed. Furthermore, various antimyocardial autoantibodies exist in the blood serum of patients with dilated cardiomyopathy. It has been revealed that such antimyocardial autoantibodies are frequently detected in patients with familial dilated cardiomyopathy (Non-patent Document 1). Furthermore, some kind of gene mutation is found in about 20% of patients with familial dilated cardiomyopathy. Most of the mutations are a mutation of gene encoding sarcomere protein directly associated with cardiac contraction and a mutation of gene encoding dystrophin-associated protein which has a role of transmitting cardiac contractile force. These gene mutations not only are a direct cause of development of dilated cardiomyopathy but also may serve as a hereditary preposition of the development. When there is such a gene mutation, a continuous autoimmune mechanism is assumed to be actuated after virus infection, and then dilated cardiomyopathy is developed. Furthermore, although patients with dilated cardiomyopathy are often a candidate for heart transplant because they have a poor prognosis, rejection following transplantation, as well as donor shortage, is a problem. For these reasons, development of a novel method for treating dilated cardiomyopathy is required.

Using animals in which cardiomyopathy was induced by a drug such as doxorubicin or 5-azacytidine as a pathological model of dilated cardiomyopathy, therapeutic effects with mesenchymal stem cells (MSC) have been studied (Non-patent Documents 2-3). However, cardiomyopathy induced by a drug such as doxorubicin or 5-azacytidine is called drug-induced cardiomyopathy, and although it is a group of cardiomyopathy clinically similar to dilated cardiomyopathy, Guidelines for Management of Dilated Cardiomyopathy and Secondary Cardiomyopathy (Guidelines for Diagnosis and Treatment of Cardiovascular Diseases 2011) classifies the disease as a disease different from dilated cardiomyopathy. Furthermore, the hereditary predisposition of dilated cardiomyopathy has been assumed to be involvement of genetic abnormalities in protein contained in the cytoplasm, such as myocardial δ-sarcoglycan, actin and myosin, and in that respect, the origin is basically different from that of drug-induced cardiomyopathy. Thus, even if mesenchymal stem cells exhibit effects of improvement in drug-induced cardiomyopathy models, it is difficult to say that they are effective in an actual dilated cardiomyopathy.

δ-Sarcoglycan deficient J2N hamsters have been reported. These hamsters have developed dilated cardiomyopathy in about 16 weeks after birth with cardiac hypofunction and morphological lesions such as hypertrophy of cardiac cells and fibrosis. These morphological lesions are similar to human lesions, and thus the hamsters have been used as a model of human dilated cardiomyopathy. Mutation of δ-sarcoglycan has also been found in human dilated cardiomyopathy; furthermore function of ANT-1 has been known to be reduced in dilated cardiomyopathy of J2N hamsters and humans (Non-patent Documents 4 to 6).

Meanwhile, mesenchymal stem cells are a multipotent precursor cell, which have been first isolated from bone marrow by Friedenstein (1982) (Non-patent Document 7). Studies have elucidated that mesenchymal stem cells exist in various tissues such as bone marrow, umbilical cord and adipose tissue. Transplantation of mesenchymal stem cells is expected as a novel method for treating various refractory diseases (Patent Documents 1 to 4). In recent years, cells having a similar function have been known to be present in stromal cells in adipose tissue or fetal appendages such as placenta, umbilical cord and egg membrane. Thus, mesenchymal stem cells are also called as mesenchymal stromal cells.

Patent Document 5 concerns a method of cell culture of an umbilical mesenchymal stem cell. Patent Document 6 concerns a particle secreted by a mesenchymal stem cell and comprising at least one biological property of a mesenchymal stem cell. Patent Document 7 concerns methods for culturing mesenchymal stem cells without the use of non-human serum components. Patent Document 8 concerns the use of exosome-preparations derived from neonatal or adult tissue-derived mesenchymal stem-cells for the prevention or for therapy of inflammatory conditions. Non-Patent Document 8 concerns the therapeutic potency of mesenchymal stem cell transplantation in cases of dilated cardiomyopathy. Non-Patent Document 9 concerns the safety, the feasibility and the efficacy of transendocardial injection of bone marrow-derived mesenchymal stem cells in patients with dilated idiopathic cardiomyopathy.

PRIOR ART DOCUMENTS

Patent Document

Patent Document 1: JP 2002-506831 A
Patent Document 2: JP 2000-508911 A
Patent Document 3: JP 2012-157263 A
Patent Document 4: JP 2012-508733 A
Patent Document 5: US 2015/0125950 A1
Patent Document 6: US 2011/0003008 A1
Patent Document 7: WO 2015/076717 A2
Patent Document 8: WO 2014/013029 A1

Non-Patent Document

Non-patent Document 1: Hitonobu Tomoike, et. al., Guidelines for Diagnosis and Treatment of Cardiovascular Diseases 2011
Non-Patent Document 2: Mol. Cell Biochem., 2014, 387, pp.279-285
Non-Patent Document 3: Med. Sci. Monit. Basic Res., 2013.14, pp.20-31
Non-Patent Document 4: Circ. J., 2005, 69, pp.107-113
Non-Patent Document 5: J. Biochem., 2003, 34(2), pp.269-76
Non-Patent Document 6: JCI., 2000, 106, pp.655-662
Non-Patent Document 7: Pittenger F. M. et al., Science, 1999.284, pp.143-147
Non-Patent Document 8: Circ., 2005, 112, pp. 1128-1135
Non-Patent Document 9: Mesenchymal Stem Cells for Idiopathic Dilated Cardiomyopathy, 8 October 2023

SUMMARY OF INVENTION

Technical Problem

The present invention was accomplished based on the above-mentioned circumstances and an object thereof is to provide an agent for use in treating dilated cardiomyopathy, which has good effects in treating dilated cardiomyopathy and achieves a consistent effect on many patients.

Solution to Problem

The present inventors conducted extensive studies to solve the above problems and have found that cardiac function and the like can be improved significantly by administering mesenchymal stem cells or microparticles derived from mesenchymal stem cells in dilated cardiomyopathy. The present invention was accomplished based on these findings. The present invention which has been accomplished to solve the above problems includes the following:

[1] An agent for use in treating dilated cardiomyopathy, comprising at least one selected from the group consisting of adipose-derived mesenchymal stem cells and microparticles derived from adipose-derived mesenchymal stem cells.
[2] The agent for use in treating dilated cardiomyopathy according to [1], wherein the mesenchymal stem cells contain microparticles or are capable of secreting microparticles.
[3] The agent for use in treating dilated cardiomyopathy according to [1] or [2], wherein the microparticles have an average particle size of 1,000 nm or less.

[4] The agent for use in treating dilated cardiomyopathy according to any one of [1] to [3], wherein the microparticles are exosome.

[5] The agent for use in treating dilated cardiomyopathy according to any one of [1] to [4], wherein the microparticles comprise at least one factor selected from the group consisting of IL-10, HGF, apolipoprotein A-2, pigment epithelium-derived factor (PEDF), SERPINF1 and haptoglobin.

[6] The agent for use in treating dilated cardiomyopathy according to any one of [1] to [5], wherein the mesenchymal stem cells are allogeneic to a subject.

[7] The agent for use in treating dilated cardiomyopathy according to any one of [1] to [6], wherein the dilated cardiomyopathy is a dilated cardiomyopathy selected from the group consisting of idiopathic dilated cardiomyopathy, familial dilated cardiomyopathy and inheritable dilated cardiomyopathy.

[8] The agent for use in treating dilated cardiomyopathy according to any one of [1] to [7], wherein the mesenchymal stem cells are cryopreserved cells.

[9] An agent for use in inhibiting fibrosis or cardiac hypertrophy in dilated cardiomyopathy, comprising at least one selected from the group consisting of adipose-derived mesenchymal stem cells and microparticles derived from adipose-derived mesenchymal stem cells.

[10] A kit for use in treating dilated cardiomyopathy, comprising the agent for use in treating dilated cardiomyopathy according to any one of [1] to [8] or the agent for use in inhibiting fibrosis or cardiac hypertrophy according to [9] a container and a label.

Advantageous Effects of Invention

According to the agent for use in treating dilated cardiomyopathy of the present invention, function, structure and the like at the area affected by diseases such as heart in dilated cardiomyopathy can be notably improved by administering adipose-derived mesenchymal stem cells or microparticles derived from adipose-derived mesenchymal stem cells. The adipose-derived mesenchymal stem cells hardly cause a rejection even to an allogeneic subject and thus donor's cells prepared in advance, which have been expanded and cryopreserved, can be used as the adipose-derived mesenchymal stem cells for the agent for use in treating dilated cardiomyopathy of the present invention. Likewise, microparticles derived from adipose-derived mesenchymal stem cells, which are donor's cells prepared in advance, which have been expanded and cryopreserved, can be used as the microparticles derived from adipose-derived mesenchymal stem cells for the agent for use in treating dilated cardiomyopathy of the present invention. For this reason, when compared with the case where autologous adipose-derived mesenchymal stem cells are prepared and used, commercialization is easier, and stable and consistent effects can be easily achieved, hence making the present invention advantageous.

BRIEF DESCRIPTION OF DRAWINGS

Fig. 1 shows therapeutic effects of the agent for use in treating dilated cardiomyopathy of the present invention on J2N-k hamsters which are an animal model of dilated cardiomyopathy (evaluation of cardiac function).

Fig. 2 shows therapeutic effects of the agent for use in treating dilated cardiomyopathy of the present invention on J2N-k hamsters which are an animal model of dilated cardiomyopathy (ATP content in the heart tissue).

Fig. 3 shows therapeutic effects of the agent for use in treating dilated cardiomyopathy of the present invention on J2N-k hamsters which are an animal model of dilated cardiomyopathy (expression levels of Mt. ANT-1 in mitochondria in the heart tissue).

Fig. 4 shows therapeutic effects of the agent for use in treating dilated cardiomyopathy of the present invention on J2N-k hamsters which are an animal model of dilated cardiomyopathy (cardiac diameter).

Fig. 5 shows therapeutic effects (long term) of the agent for use in treating dilated cardiomyopathy of the present invention on J2N-k hamsters which are an animal model of dilated cardiomyopathy (evaluation of cardiac function).

Fig. 6 shows effects of the agent for use in treating dilated cardiomyopathy of the present invention for increasing function of human cardiomyocytes.

DESCRIPTION OF EMBODIMENTS

Hereinafter, the agent for treating dilated cardiomyopathy, the inhibitor of fibrosis, the inhibitor of cardiac hypertrophy and the kit for treating dilated cardiomyopathy for use according to the present invention will be described in detail.

<Agent for use in treating dilated cardiomyopathy>

The agent for use in treating dilated cardiomyopathy of the present invention contains at least one selected from the group consisting of adipose-derived mesenchymal stem cells and microparticles derived from adipose-derived mesenchymal stem cells.

<Mesenchymal stem cells>

Mesenchymal stem cells in the present invention means cells having differentiation potency into cells belonging to the mesenchymal (bone cells, cardiac muscle cells, chondrocytes, tendon cells, adipocytes, etc.) and capable of proliferating while maintaining such a potency. The term mesenchymal stem cells used in the present invention means cells which are the same as stromal cells and do not particularly distinguish both from each other. Examples of the tissue containing mesenchymal stem cells include adipose tissue, umbilical cord, bone marrow, umbilical-cord blood, endometrial, placenta, dermis, skeletal muscle, periosteum, dental sac, periodontal ligament, pulp, and tooth-germ. Thus, an adipose-derived mesenchymal stem cells mean, for example, mesenchymal stem cells contained in an adipose tissue and may be termed as adipose-derived stromal cells. From the viewpoints of effects to ameliorate visceral diseases and the like by the agent for use in treating dilated cardiomyopathy of the present invention and availability, adipose-derived mesenchymal stem cells are used. Umbilical cord-derived mesenchymal stem cells, bone marrow-derived mesenchymal stem cells, placenta-derived mesenchymal stem cells, and pulp-derived mesenchymal stem cells described herein are not part of the invention.

Mesenchymal stem cells in the present invention are preferably autologous or allogeneic to a subject. The mesenchymal stem cells hardly cause a rejection even to an allogeneic subject and thus donor's cells prepared in advance are expanded, cryopreserved and can be used as the mesenchymal stem cells for the agent for treating dilated cardiomyopathy of the present invention. For this reason, when compared with the case where autologous mesenchymal stem cells are prepared and used, the mesenchymal stem cells in the present invention are more preferably allogenic from the viewpoint that commercialization is easier and stable and consistent effects can be easily achieved.

The mesenchymal stem cells hardly cause a rejection even to an allogeneic subject and thus donor's cells prepared in advance, which have been expanded and cryopreserved, can be used as the mesenchymal stem cells for the agent for treating dilated cardiomyopathy of the present invention. Likewise, microparticles derived from mesenchymal stem cells, which are donor's cells prepared in advance, which have been expanded and cryopreserved, can be used as the microparticles derived from mesenchymal stem cells for the agent for treating dilated cardiomyopathy of the present invention. For this reason, when compared with the case where autologous mesenchymal stem cells or microparticles are prepared and used, the mesenchymal stem cells in the present invention are more preferably allogenic from the viewpoint that commercialization is easier and stable and consistent effects can be easily achieved.

Mesenchymal stem cells in the present invention also mean any cell populations containing mesenchymal stem cells. At least 20% or more, preferably 30%, 40%, 50%, 60%, 70%, 75%, 80%, 85%, 90%, 93%, 96%, 97%, 98% or 99% of such a cell population consists of mesenchymal stem cells.

Adipose tissue in the present invention means a tissue containing stromal cells containing adipocytes and microvascular cells and, for example, tissues obtained by surgical resection or aspiration from subcutaneous fat of a mammal. Adipose tissue can be obtained from subcutaneous fat. Subcutaneous fat is preferably obtained from a species same as a subject to administration of adipose-derived mesenchymal stem cells to be described later, and when considering administration to human, human subcutaneous fat is more preferable. A subcutaneous fat-supplying individual may be alive or dead, but adipose tissue used in the present invention is preferably tissues collected from a live individual. In the case of collecting from an individual, examples of the liposuction include PAL (Power-Assisted) Liposuction, elcornia laser liposuction, and body jet liposuction, and from a viewpoint of preserving the condition of cells, ultrasonic wave is preferably not used.

Umbilical cord in the present invention is a white conduit tissue connecting a fetus and the placenta, composed of umbilical vein, umbilical artery, mucous connective tissue (Wharton's Jelly), and umbilical substrate itself and containing a large number of mesenchymal stem cells. Umbilical cord is preferably obtained from a species same as a subject (subject to administration) to whom the agent for use in treating dilated cardiomyopathy is used, and when considering administration of the agent for treating dilated cardiomyopathy to human, human umbilical cord is more preferable.

Bone marrow in the present invention refers to a parenchyma filling the bone lumen and is a hematopoietic organ. Bone marrow fluid is present in the bone marrow, and the cells present therein are called bone marrow cells. Bone marrow cells contain, in addition to red-blood cells, granulocytes, megakaryocytes, lymphocytes, and adipocytes, mesenchymal stem cells, hematopoietic stem cells, vascular endothelial precursor cells and the like. Bone marrow cells can be collected from, for example, human iliac, long bone, or other bones.

Various tissues-derived mesenchymal stem cells such as adipose-derived mesenchymal stem cells, umbilical cord-derived mesenchymal stem cells, and bone marrow-derived mesenchymal stem cells in the present invention mean any cell populations containing each tissue-derived mesenchymal stem cells such as adipose-derived mesenchymal stem cells, umbilical cord-derived mesenchymal stem cells, and bone marrow-derived mesenchymal stem cells, respectively. At least 20% or more, preferably 30%, 40%, 50%, 60%, 70%, 75%, 80%, 85%, 90%, 93%, 96%, 97%, 98% or 99% of such a cell population consists of each tissue-derived mesenchymal stem cells such as adipose-derived mesenchymal stem cells, umbilical cord-derived mesenchymal stem cells, and bone marrow-derived mesenchymal stem cells, respectively.

The mesenchymal stem cells in the present invention may be characterized by, for example, growth characteristics (e.g., population doubling capability and doubling time from passage to senescence), karyotype analysis (e.g., normal karyotype, maternal or neonatal lineage), surface marker expression in flow cytometry (e.g., FACS analysis), immunohistochemistry and/or immunocytochemistry (e.g., detection of epitope), gene expression profiling (e.g., gene chip arrays; polymerase chain reaction such as reverse transcription PCR, real-time PCR and conventional PCR), miRNA expression profiling, protein arrays, secretion of protein such as cytokine (e.g., plasma coagulation assay, ELISA, cytokine arrays), metabolites (metabolome analysis) and other methods known in the art.

<Method for preparing mesenchymal stem cells>

The mesenchymal stem cells can be prepared by a method well known by a person skilled in the art. A method for preparing adipose-derived mesenchymal stem cells is described below as an example. Adipose-derived mesenchymal stem cells may be obtained by the production method described in, for example, U.S. Pat No. 6,777,231, and can be produced by a method comprising, for example, the following steps (i) to (iii):

(i) a step of obtaining a cell suspension by enzymatically digesting adipose tissue;
(ii) a step of sedimenting cells and resuspending in suitable medium; and
(iii) a step of culturing cells on a solid surface and removing cells not binding to the solid surface.

For the adipose tissue used in the step (i), it is preferable to use those which have been washed. Washing can be carried out using a physiologically compatible saline solution (for example, phosphate buffer solution (PBS)) by vigorously stirring to cause sedimentation. This is to remove impurities (also called debris including, for example, damaged tissue, blood, and red-blood cells) contained in adipose tissue therefrom. For this reason, washing and sedimentation are repeated in general until all debris is removed from the supernatant. Remaining cells are present in the form of masses of different sizes, and for this reason it is preferable for washed cell masses to have cell junctions weaken or enzymatically treated for destruction (for example, collagenase, dispase, or trypsin) to dissociate while minimizing damages of the cells themselves. An amount of and a treatment period with an enzyme vary depending on conditions employed but are already known in the technical field concerned. In place of or in combination with such an enzymatic treatment, cell masses can be decomposed by other treatment methods such as mechanical stirring, ultrasonic energy, or thermal energy but it is preferable to carry out only by enzymatic treatment for minimizing cell damages. When an enzyme is used, it is desirable to deactivate the enzyme using medium or the like after taking a suitable period for minimizing a harmful action on cells.

The cell suspension obtained in the step (i) contains a slurry or a suspension of aggregated cells and various contaminant cells such as red-blood cells, smooth-muscle cells, endothelial cells, and fibroblast. Accordingly, the aggregated cells and these contaminant cells may subsequently be separated and removed but the removal can be achieved by adhering and washing in the step (iii) to be described later whereby such a separation and removal may be omitted. The separation and removal of contaminant cells, if performed, can be achieved by centrifugation in which cells are forcibly separated into the supernatant and the precipitation. The obtained precipitate containing contaminant cells is suspended in a physiologically compatible solvent. Suspended cells are likely to contain red-blood cells but a step of dissolution is not always necessary because red-blood cells are excluded by the selection of adhering to a solid surface to be described later. As a method for selectively dissolving red-blood cells, a method well known in the technical field concerned can be employed such as incubation in hypertonic medium or hypotonic medium by the dissolution in ammonium chloride. After dissolution, the lysate may be separated from desirable cells by, for example, filtration, centrifugal sedimentation, or density fraction.

In the step (ii), the suspended cells may be washed once or several times consecutively for enhancing the purity of mesenchymal stem cells, centrifuged, and resuspended in medium. In addition to this, the cells may be separated based on cell surface markers profile or on the size and granular properties.

Medium used for resuspension is not particularly limited as long as it can culture mesenchymal stem cells, and such a medium may be created by adding, to basal medium, serum and/or one or more serum substitutes such as albumin, transferrin, fatty acid, insulin, sodium selenite, cholesterol, a collagen precursor, a trace element, 2-mercaptoethanol, and 3'- thioglycerol. These medium may further contain substances, as necessary, such as lipid, amino acid, protein, polysaccharide, vitamin, growth factor, low molecular weight compound, antibiotic, antioxidant, pyruvate, buffer, and inorganic salts. Example of the basal medium include IMDM, Medium 199, Eagle's Minimum Essential Medium (EMEM), αMEM, Dulbecco's modified Eagle's Medium (DMEM), Ham's F12 medium, RPMI 1640 medium, Fischer's medium, MCDB201 medium, and combinations of these media. Examples of the serum includes, but not limited to, human serum, fetal bovine serum (FBS), bovine serum, bovine calf serum, goat serum, horse serum, porcine serum, sheep serum, rabbit serum, and rat serum. Serum, when used, may be added in 5 v/v% to 15 v/v%, preferably 10 v/v%, to the basal medium. Examples of the fatty acid include, but not limited to, linoleic acid, oleic acid, linolenic acid, arachidonic acid, myristic acid, palmitoyl acid, palmitic acid, and stearic acid. Examples of the lipid include, but not limited to, phosphatidyl serine, phosphatidylethanolamine, and phosphatidylcholine. Examples of the amino acid include, but not limited to, L-alanine, L-arginine, L-aspartic acid, L-asparagine, L-cysteine, L-cystine, L-glutamic acid, L-glutamine, and L-glycine. Examples of the protein include, but not limited to, ecotin, reduced glutathione, fibronectin, and $\beta$2-microglobulin. Examples of the polysaccharide include glycosaminoglycan, of which examples particularly include, but not limited to, hyaluronic acid and heparan sulfate. Examples of the growth factor include, but not limited to, platelet-derived growth factor (PDGF), basic fibroblast growth factor (bFGF), transforming growth factor-$\beta$ (TGF-$\beta$), hepatocyte growth factor (HGF), epidermal growth factor (EGF), connective tissue growth factor (CTGF), and vascular endothelial growth factor (VEGF). Xeno-free medium, which does not contain xeno-derived components such as serum, is preferably used from the viewpoint of using the adipose-derived mesenchymal stem cells obtained in the present invention for cell transplantation. These media are available in the form of pre-prepared medium for mesenchymal stem cells (stromal cells) from, for example, PromoCell, Lonza, Biological Industries, Veritas Corporation, R&D Systems, Corning Inc., and Rohto Pharmaceutical Co., Ltd.

Subsequently, in the step (iii), cells in the cell suspension obtained in the step (ii) are cultured without being differentiated on a solid surface using the above-mentioned suitable cell medium under a suitable cell density and culture conditions. "Solid surface" in the present invention means any material which enables the binding of the adipose-derived mesenchymal stem cells of the present invention. In a specific embodiment, such a material is a plastic material treated to facilitate the binding of mammalian cells to the surface thereof. The shape of a culture vessel with a solid surface is not limited but a petri dish and a flask are preferably used. Cells are washed after incubation to remove non-binding cells and cell debris.

In the present invention, cells which finally stay in a binding state on a solid surface can be selected as a cell population of adipose-derived mesenchymal stem cells.

The selected cells may be analyzed on the surface antigens by a conventional method using flow cytometry or the like for confirming that they are adipose-derived mesenchymal stem cells of the present invention. Additionally, the cells may be tested on the differentiation potency into each cell line, and such a differentiation can be carried out by a conventional method.

Mesenchymal stem cells in the present invention can be prepared as described above but may be defined as the cells having the following characteristics;

(1) adhesive property to plastic is demonstrated under culture conditions in standard medium,
(2) surface antigens CD44, CD73, and CD90 are positive, and CD31 and CD45 are negative, and
(3) differentiations into bone cells, adipocytes, and chondrocytes are feasible under culture conditions.

<Cryopreservation of mesenchymal stem cells>

The mesenchymal stem cells in the present invention may be cells repeatedly cryopreserved and melted as necessary as long as having disease treatment effects. Cryopreservation in the present invention can be carried out by suspending mesenchymal stem cells in a cryopreservation solution well known by a person skilled in the art and cooling it. The suspension can be carried out by dissociating the cells using a dissociating agent such as trypsin, moving to a cryopreservation container, treating as necessary, and subsequently adding a cryopreservation solution thereto.

A cryopreservation solution may contain DMSO (Dimethyl sulfoxide) as a cryoprotective agent but DMSO has characteristics which induces differentiation of mesenchymal stem cells in addition to cytotoxicity and hence it is preferable to reduce a content of DMSO. Examples of the DMSO substitute include glycerol, propylene glycol, and polysaccharides. DMSO, when used, is contained in 5 v/v% to 20 v/v%, preferably 5 v/v% to 10 v/v%, and more preferably 10 v/v%. Additionally, additives described in WO2007/058308 may be contained. For such a cryopreservation solution, cryopreservation solutions provided by for example, BioVerde, NIPPON Genetics Co, Ltd, ReproCELL Inc., ZENOAQ, COSMO BIO, Kohjin Bio Co., Ltd., and Thermo Fisher Scientific may be used.

The above-mentioned suspended cells, when cryopreserved, may be stored at a temperature between -80°C to -100°C (for example, -80°C), and cryopreservation can be carried out using any freezer capable of achieving such a temperature. For avoiding abrupt temperature changes, a cooling rate may be controlled suitably using a programmed freezer but not particularly limited thereto. Cooling rate may suitably be selected depending on components of a cryopreservation solution and can be done according to a direction of a cryopreservation solution manufacturer.

Storage duration is not particularly limited in the upper limit as long as melted cells cryopreserved under the above conditions retain properties equal to before frozen, and examples include 1 week or more, 2 weeks or more, 3 weeks or more, 4 weeks or more, 2 months or more, 3 months or more, 4 months or more, 5 months or more, 6 months or more, 1 year or more, and more than these. Cell damages can be suppressed when stored at a lower temperature, and thus the cells may be moved to a gas phase on liquid nitrogen (about -150°C or lower to -180°C or higher) and stored therein. Storage, when performed in a gas phase on liquid nitrogen, can be carried out using a preservation container well known by a person skilled in the art. For example, in the case of storing for 2 weeks or more, but not limited thereto, it is preferable to store in a gas phase on liquid nitrogen.

Melted mesenchymal stem cells may suitably be cultured until next cryopreservation. Culture of mesenchymal stem cells is carried out using the above-mentioned medium capable of culturing mesenchymal stem cells, and may be carried out at a culture temperature of about 30 to 40°C, and preferably about 37°C in an atmosphere of air containing $CO_2$, but not limited thereto. A $CO_2$ concentration is about 2 to 5%, and preferably about 5%. During culture, upon reaching a suitable confluency to a culture vessel (examples include cells occupying 50% to 80% of a culture vessel), the cells are dissociated using a dissociating agent such as trypsin and sown into a culture vessel separately provided in a suitable cell density to continue culture. When inoculating cells, typical examples of the cell density include about 100 cells/cm$^2$ to about 100,000 cells/cm$^2$, about 500 cells/cm$^2$ to about 50,000 cells/cm$^2$, about 1,000 to 10,000 cells/cm$^2$, and about 2,000 to 10,000 cells/cm$^2$. In a specific embodiment, a cell density is 2,000 to 10,000 cells/cm$^2$. It is preferable to adjust a period before a suitable confluency is reached in such a way as to be 3 days to 7 days. During culture, medium may suitably be replaced as necessary.

The cryopreserved cells can be melted using a method well known by a person skilled in the art. Examples include a method in which the cells are allowed to stand or shaken in a thermostatic chamber or a hot water bath at 37°C.

<Microparticles derived from mesenchymal stem cells>

The microparticles derived from mesenchymal stem cells in the present invention refer to microparticles obtained from mesenchymal stem cells, or produced by mesenchymal stem cells. The microparticles derived from mesenchymal stem cells in the present invention may be contained in mesenchymal stem cells or in a culture supernatant of mesenchymal stem cells, or may be isolated from mesenchymal stem cells or a culture supernatant of mesenchymal stem cells. In other words, the microparticles derived from mesenchymal stem cells in the present invention may be in any form. They may be not only in the form of microparticles themselves but also in the form of mesenchymal stem cells containing microparticles or capable of secreting microparticles.

Methods of isolating microparticles include ultracentrifugation, microfiltration, capturing using antibody and utilization of a microfluid system. Microparticles isolated may contain cells such as mesenchymal stem cells or a culture medium of mesenchymal stem cells.

The microparticles derived from mesenchymal stem cells in the present invention may be collected from a culture supernatant obtained by culturing mesenchymal stem cells using the above medium. When collecting the culture supernatant, mesenchymal stem cells are, for example, brought to the state of being sub-confluent or confluent in a culture container, transferred to a new medium, and then further cultured for 1 to 5 days, and the culture supernatant may be collected. This culture supernatant may be used as the agent for treating dilated cardiomyopathy of the present invention, or microparticles may be separated by ultrafiltration, density gravity centrifugation, various types of kits for separating microparticles or the like, and the microparticles may be used as a material of the agent for treating dilated cardiomyopathy of the present invention.

The agent for use in treating dilated cardiomyopathy of the present invention may contain, in addition to the above microparticles, mesenchymal stem cells themselves which contain the above microparticles or are capable of secreting the microparticles. When the agent for use in treating dilated cardiomyopathy of the present invention contains mesenchymal stem cells which contain the above microparticles or are capable of secreting the microparticles, containing the above mesenchymal stem cells may be understood to satisfy the requirement of containing the above microparticles simultaneously.

The microparticles derived from mesenchymal stem cells in the present invention are typically a vesicle released from mesenchymal stem cells and having such a size that can be observed with an electron microscope. For the size of the microparticles, the microparticles have an average particle size of 1 nm or more and 1,000 nm or less, preferably 10 nm or more and 500 nm or less, and further preferably 30 nm or more and 200 nm or less. Here, the average particle size means an average value of the diameter of particles measured by a dynamic light scattering method or with an electron microscope. The above microparticles may have a lipid bilayer surrounding biomolecules.

Examples of microparticles described above include membrane particles, membrane vesicles, microvesicles, nanovesicles, microvesicles (having an average particle size of 30 to 1,000 nm), exosome-like vesicles, exosome (having an average particle size of 30 to 200 nm), ectosome-like vesicles, ectosome and exovesicles, and exosome is preferred. Different types of microparticles derived from mesenchymal stem cells are also distinguished by cellular origin, the density, shape and rate of sedimentation of microparticles in sucrose, the composition of lipid, protein markers, and the system of secretion (i.e., after signal (signal-induced) or spontaneous (constitutive)). Those microparticles are fractionated, for example, by a density gravity centrifugation, into a fraction of 1.0 to 1.5 g/mL, and preferably 1.1 to 1.3 g/mL. The microparticles contain any one of phosphatidylserine, cholesterol, sphingomyelin and ceramide as lipid constituting the particle.

The microparticles derived from mesenchymal stem cells in the present invention contain protein, fatty acid and nucleic acid such as miRNA. Examples of protein and fatty acid described above include IL-10, HGF (Hepatocyte Growth Factor), apolipoprotein A-2, pigment epithelium-derived factor (PEDF), SERPINF1, haptoglobin, pelargonic acid, lauric acid, myristic acid, pentadecanoic acid, isopentadecanoic acid, palmitic acid, isopalmitic acid, margaric acid, isoheptadecanoic acid, stearic acid, isostearic acid, oleic acid, elaidic acid, linoleic acid, nonadecylic acid, isononadecylic acid, arachidic acid, 11Z-eicosenoic acid, dihomo-$\gamma$-linolenic acid, arachidonic acid, erucic acid, 13Z, 16Z-Docosadienoic acid, 13Z, 16Z, 19Z-Docosadienoic acid, adrenic acid, clupanodonic acid, preferably IL-10, HGF, apolipoprotein A-2, pigment epithelium-derived factor (PEDF), SERPINF1, haptoglobin, pelargonic acid, lauric acid, myristic acid, pentadecanoic acid, isopentadecanoic acid, palmitic acid, isopalmitic acid, margaric acid, isoheptadecanoic acid, stearic acid, isostearic acid, oleic acid, elaidic acid, linoleic acid, arachidic acid, 11Z-eicosenoic acid, arachidonic acid, erucic acid, 13Z,16Z-Docosadienoic acid, further preferably IL-10, HGF, apolipoprotein A-2, pigment epithelium-derived factor (PEDF), SERPINF1, haptoglobin, myristic acid, pentadecanoic acid, palmitic acid, margaric acid, isoheptadecanoic acid, stearic acid, oleic acid, linoleic acid, nonadecylic acid, erucic acid, 13Z,16Z-Docosadienoic acid, and particularly preferably IL-10, HGF, palmitic acid, margaric acid, stearic acid, oleic acid and 13Z,16Z-Docosadienoic acid. It is preferable that the microparticles derived from mesenchymal stem cells in the present invention are microparticles containing the above.

<Form of mesenchymal stem cells and microparticles derived from mesenchymal stem cells>

Mesenchymal stem cells of the present invention may be a cell in any condition such as a cell dissociated during culture and collected or a cell in a frozen condition in a cryopreservation solution. Same lots of cells obtained by expansion subdivided and cryopreserved are preferably used from the viewpoint of obtaining stable effects and good handleability. The cryopreserved mesenchymal stem cells may be melted immediately before use and, may be directly administered while being suspended in a cryopreservation solution, or may be administered after suspended in an infusion or medium. Alternatively, the cells may be mixed with an infusion or medium after a cryopreservation solution is removed by a method such as centrifugation.

The microparticles derived from mesenchymal stem cells in the present invention may be a maicroparticle in any condition such as microparticles derived from mesenchymal stem cells isolated from cells during culture, or microparticles derived from mesenchymal stem cells in a frozen condition in a cryopreservation solution. It is preferable to use microparticles obtained by subdividing and cryopreserving microparticles derived from mesenchymal stem cells which have been isolated from same lots of cells obtained by expansion, because, for example, stable effects are obtained and handleability is excellent. The cryopreserved and isolated microparticles derived from mesenchymal stem cells may be melted immediately before use and, may be directly administered while being suspended in a cryopreservation solution, or may be administered after suspended in an infusion or medium. Alternatively, the microparticles may be mixed with an infusion or medium after a cryopreservation solution is removed by a method such as centrifugation.

"Infusion" in the present invention herein refers to a solution used when treating human, and examples include, but not limited to, a physiological saline solution, a Japanese pharmacopoeia physiological saline, a 5% glucose solution, a Japanese pharmacopoeia glucose injection, a Ringer's solution, a Japanese pharmacopoeia Ringer's solution, a lactated Ringer's solution, an acetate Ringer's solution, No. 1 solution (starting solution), No. 2 solution (rehydration solution), No. 3 solution (maintenance solution), and No. 4 solution (postoperative recovery solution). The above infusion, medium and the like may be prepared so that they contain other components (pharmacologically acceptable carriers and additives) described later.

The agent for use in treating dilated cardiomyopathy of the present invention may contain pharmacologically acceptable carriers and additives according to a routine method depending on the purpose of usage and form thereof within the range in which the effects of the present invention are not affected. Examples of such carriers and additives include tonicity adjusting agents, thickeners, saccharides, sugar alcohols, antiseptics (preservatives), bactericides or antimicrobial agents, pH adjusters, stabilizers, chelating agents, oleaginous bases, gel bases, surfactants, suspending agents, binders, excipients, lubricants, disintegrants, foaming agents, fluidizing agents, dispersants, emulsifiers, buffers, solubilizing agents, antioxidants, sweeteners, acidulating agents, colorants, flavoring agents, perfumes, and cooling agents, but not limited thereto.

The agent for use in treating dilated cardiomyopathy of the present invention may be provided in various forms including dosage forms such as a solid preparation, a semi-solid preparation and a liquid depending on the purpose. The agent is available in the form of a solid preparation (e.g., tablets, powders, powdered preparations, granules and capsules), a semi-solid preparation (e.g., ointments such as hard ointments and soft ointments, and cream), a liquid (e.g., lotion, extract, suspension, emulsion, syrup, injection (including infusion, implant injection, sustained injection and injection prepared before use), dialysis agents, aerosol, soft capsules and drinks), a patch, or a poultice. The agent for use in treating dilated cardiomyopathy of the present invention is also available in the form of a solution, an emulsion or the like in an oily or aqueous vehicle. Furthermore, the agent for use in treating dilated cardiomyopathy of the present invention may be applied to an affected area by spraying, and thus is also available in the form in which the agent is gelled or formed into sheet at the affected area after spraying. Alternatively, the above mesenchymal stem cells may be formed into a sheet or a three-dimensional structure, and then the agent for use in treating dilated cardiomyopathy of the present invention may be applied to the affected area.

The agent for use in treating dilated cardiomyopathy of the present invention may be used after being suspended or diluted using infusion such as a physiological saline solution, a Japanese pharmacopoeia physiological saline, a 5% glucose solution, a Japanese pharmacopoeia glucose injection, a Ringer's solution, a Japanese pharmacopoeia Ringer's solution, a lactated Ringer's solution, an acetate Ringer's solution, a bicarbonate Ringer's solution, No. 1 solution (starting solution), No. 2 solution (rehydration solution), No. 3 solution (maintenance solution), and No. 4 solution (postoperative recovery solution), or a cell culture medium such as DMEM. The agent may be used after being suspended or diluted using, preferably a physiological saline solution, a 5% glucose solution, or No. 1 solution (starting solution), and more preferably a 5% glucose solution or No. 1 solution (starting solution).

When the agent for use in treating dilated cardiomyopathy of the present invention is in the form of a liquid, the pH of the agent for treating dilated cardiomyopathy is not particularly limited as long as it is within the pharmacologically (pharmaceutically) or physiologically acceptable range. The pH is, for example, in the range of 2.5 to 9.0, preferably 3.0 to 8.5, and more preferably 3.5 to 8.0.

When the agent for use in treating dilated cardiomyopathy of the present invention is in the form of a liquid, the osmotic pressure of the agent for treating dilated cardiomyopathy is not particularly limited as long as it is acceptable for living organisms. The osmotic pressure ratio of the composition of the present invention is, for example, in the range of preferably 0.7 to 5.0, more preferably 0.8 to 3.0, and further preferably 0.9 to 1.4. The osmotic pressure may be adjusted using inorganic salt, polyhydric alcohol, sugar alcohol, saccharide or the like, by a method known in the art. The osmosis pressure ratio is defined as the ratio of the osmosis pressure of a sample to the osmosis pressure of 286 mOsm (0.9 w/v% aqueous sodium chloride solution) in accordance with the Japanese Pharmacopoeia, Fifteenth Edition. The osmosis pressure is measured by the method for measuring osmolarity (freezing point depression method) described in the Japanese Pharmacopoeia. The standard solution for measuring osmosis pressure ratios (0.9 w/v% aqueous sodium chloride solution) is prepared by drying sodium chloride (Japanese Pharmacopoeia standard reagent) at 500 to 650°C for 40 to 50 minutes, then being left to cool in a desiccator (silica gel), precisely weighing 0.900 g thereof, and dissolving it in purified water to give exactly 100 mL of solution. Alternatively, a commercially available standard solution for measuring osmosis pressure ratios (0.9 w/v% aqueous sodium chloride solution) is used.

The agent for use in treating dilated cardiomyopathy of the present invention may be in the form such that mesenchymal stem cells or microparticles derived from mesenchymal stem cells and a solution for suspending mesenchymal stem cells or microparticles derived from mesenchymal stem cells are sealed and stored in an individual container, and both are mixed when in use. When stored, the above mesenchymal stem cells or microparticles derived from mesenchymal stem cells and the solution for suspending mesenchymal stem cells or microparticles derived from mesenchymal stem cells may be frozen or refrigerated.

The agent for use in treating dilated cardiomyopathy of the present invention can be used for treating, preferably dilated cardiomyopathy, and more preferably inheritable dilated cardiomyopathy, familial dilated cardiomyopathy and idiopathic dilated cardiomyopathy.

Examples of routes of administration of the agent for use in treating dilated cardiomyopathy of the present invention include direct administration to the surface of the heart, intracardial administration, oral administration, subcutaneous administration, intramuscular administration, intravenous administration, intra-arterial administration, intraspinal administration, sublingual administration, rectal administration, vaginal administration, nasal administration, inhalation and percutaneous administration. The route is preferably direct administration to the surface of the heart, intramuscular administration, intravenous administration and intra-arterial administration from the viewpoint of the effectiveness of the agent for treating dilated cardiomyopathy of the present invention.

The dose (dosage) of mesenchymal stem cells of the agent for use in treating dilated cardiomyopathy of the present invention may vary depending on the patient's conditions (body weight, age, symptoms, physical conditions and the like) and the dosage form of the agent for treating dilated cardiomyopathy of the present invention. The dose of mesenchymal stem cells is preferably high in order to produce a sufficient therapeutic effect of the agent for treating dilated cardiomyopathy, and the dose is preferably low in order to prevent the occurrence of side effects. Typically, for administration (spraying) to an adult, a dose is $1 \times 10^3$ to $1 \times 10^{12}$ cells/dose, preferably $1 \times 10^4$ to $1 \times 10^{11}$ cells/dose, more preferably $1 \times 10^5$ to $1 \times 10^{10}$ cells/dose, and further preferably $5 \times 10^6$ to $1 \times 10^9$ cells/dose in terms of the number of cells. Furthermore, the dose (spraying) of mesenchymal stem cells is $1 \times 10$ to $5 \times 10^{10}$ cells/kg, preferably $1 \times 10^2$ to $5 \times 10^9$ cells/kg, more preferably $1 \times 10^3$ to $5 \times 10^8$ cells/kg, and further preferably $1 \times 10^4$ to $5 \times 10^7$ cells/kg based on the weight of a patient. The above dose of mesenchymal stem cells may be administered (sprayed) as a single dose several times, or may be administered (sprayed) in several divided doses.

When the agent for use in treating dilated cardiomyopathy of the present invention contains mesenchymal stem cells which secrete microparticles, its dose (dosage) may vary depending on the patient's conditions (body weight, age, symptoms, physical conditions and the like) and the dosage form of the agent for treating dilated cardiomyopathy of the present invention. The dose is preferably high in order to produce a sufficient therapeutic effect of the agent for treating dilated cardiomyopathy, and the dose is preferably low in order to prevent the occurrence of side effects. Typically, for administration to an adult, a dose is $1 \times 10^3$ to $1 \times 10^{12}$ cells/dose, preferably $1 \times 10^4$ to $1 \times 10^{11}$ cells/dose, more preferably $1 \times 10^5$ to $1 \times 10^{10}$ cells/dose, and particularly preferably $5 \times 10^6$ to $1 \times 10^9$ cells/dose in terms of the number of cells. The above dose may be administered as a single dose several times, or may be administered in several divided doses.

When the agent for use in treating dilated cardiomyopathy of the present invention contains mesenchymal stem cells which secrete microparticles, its dose (dosage) may vary depending on the patient's conditions (body weight, age, symptoms, physical conditions and the like) and the dosage form of the agent for treating dilated cardiomyopathy of the present invention. Typically, for administration to an adult, a dose is $1 \times 10$ to $5 \times 10^{10}$ cells/kg, preferably $1 \times 10^2$ to $5 \times 10^9$ cells/kg, more preferably $1 \times 10^3$ to $5 \times 10^8$ cells/kg, and particularly preferably $1 \times 10^4$ to $5 \times 10^7$ cells/kg in terms of the number of cells. Note that the above dose, as a single dose, may be administered several times or may be administered in several divided doses.

When the agent for use in treating dilated cardiomyopathy of the present invention contains isolated microparticles or a culture supernatant of mesenchymal stem cells containing microparticles, its dose (dosage) may vary depending on the patient's conditions (body weight, age, symptoms, physical conditions and the like) and the dosage form of the agent for treating dilated cardiomyopathy of the present invention. Typically, for administration to an adult, the dose of microparticles is $1 \times 10^4$ to $5 \times 10^{13}$ particles/kg, preferably $1 \times 10^5$ to $5 \times 10^{12}$ particles/kg, more preferably $1 \times 10^6$ to $5 \times 10^{11}$ particles/kg, and particularly preferably $1 \times 10^7$ to $5 \times 10^{10}$ particles/kg. The above dose may be administered as a single dose several times, or may be administered in several divided doses.

The agent for use in treating dilated cardiomyopathy of the present invention may be administered together with one or two or more other agent. Other agents include any agent that can be used as an agent for treating dilated cardiomyopathy, a heart disease. Examples thereof include ACE inhibitors, angiotensin II receptor antagonists, β blockers, antiplatelet drugs, warfarin, calcium channel blockers, nitrates, diuretics, HMG-CoA reductase inhibitors and Ancaron. Administering the agent for use in treating dilated cardiomyopathy of the present invention together with one or two or more other agent includes various cases, e.g., administering the agent for use in treating dilated cardiomyopathy of the present invention simultaneously with another agent, administering either one of them and then the other agent after a certain time, and a combination thereof.

The agent for use in treating dilated cardiomyopathy of the present invention has a significant therapeutic effect on subjects who have contracted dilated cardiomyopathy. The above therapeutic effect can be confirmed by, for example, echocardiography or histopathological examination (examination of cardiac hypertrophy and fibrosis). More specifically, the agent for use in treating dilated cardiomyopathy of the present invention can suppress cardiac hypertrophy and fibrosis of the cardiac tissue, and thus can improve cardiac function of patients who have contracted dilated cardiomyopathy. Furthermore, the effect can be maintained for a long time. Moreover, the agent for use in treating dilated cardiomyopathy of the present invention contributes to increase ATP content and expression levels of ANT-1 protein in mitochondria in the heart tissue of patients who have contracted dilated cardiomyopathy and thus can increase survival.

<Method for preparing agent for treating dilated cardiomyopathy>

The agent for use in treating dilated cardiomyopathy of the present invention is prepared by mixing mesenchymal stem cells or microparticles derived from mesenchymal stem cells and an appropriate liquid and the like for suspending cells or microparticles (containing a pharmacologically acceptable carrier or an additive) depending on each dosage form according to a routine method. A preferable example is a method for preparing an agent for treating dilated cardiomyopathy in the form of gel by suspending mesenchymal stem cells or microparticles derived from mesenchymal stem cells in a fibrinogen solution and directly spraying the resulting suspension and a thrombin solution to an area affected by diseases substantially simultaneously, or by suspending mesenchymal stem cells or microparticles derived from mesenchymal stem cells in a thrombin solution and directly spraying the resulting suspension and a fibrinogen solution to an area affected by diseases substantially simultaneously.

<Inhibitor of fibrosis>

The present invention also includes an inhibitor of fibrosis in dilated cardiomyopathy, comprising mesenchymal stem cells or microparticles derived from mesenchymal stem cells. The agent for use in treating dilated cardiomyopathy of the present invention described above has a significant effect of suppressing, in particular, fibrosis in dilated cardiomyopathy, and thus is also effective as an inhibitor of fibrosis in dilated cardiomyopathy. The above description of the agent for treating dilated cardiomyopathy is applicable to the inhibitor of fibrosis in dilated cardiomyopathy of the present invention.

<Inhibitor of cardiac hypertrophy>

The present invention also includes an inhibitor of cardiac hypertrophy in dilated cardiomyopathy, containing mesenchymal stem cells or microparticles derived from mesenchymal stem cells. The agent for use in treating dilated cardiomyopathy of the present invention described above has a significant effect of suppressing, in particular, cardiac hypertrophy in dilated cardiomyopathy, and thus is also effective as an inhibitor of cardiac hypertrophy in dilated cardiomyopathy. The above description of the agent for treating dilated cardiomyopathy is applicable to the inhibitor of cardiac hypertrophy in dilated cardiomyopathy of the present invention.

<Kit for treating dilated cardiomyopathy>

The present invention also includes a kit for treating dilated cardiomyopathy, containing the agent for treating dilated cardiomyopathy, the inhibitor of fibrosis, or the inhibitor of cardiac hypertrophy described above, a container and a label. Examples of suitable containers that the kit of the present invention contains include, but are not limited to, a cryotube for freezing mesenchymal stem cells or microparticles derived from mesenchymal stem cells, a bottle, a vial or a test tube for a liquid suspending mesenchymal stem cells or microparticles derived from mesenchymal stem cells. These containers may be made of various materials such as glass, metal and plastic, or a combination thereof. The labels on these containers describes the content.

<Method for treatment>

In an embodiment, not part of the invention, it is disclosed a method for treating dilated cardiomyopathy, comprising administering at least one selected from the group consisting of mesenchymal stem cells and microparticles derived from mesenchymal stem cells. According to the method for treating dilated cardiomyopathy, function, structure and the like at the area affected by diseases such as heart in the patient of dilated cardiomyopathy can be notably improved by administering mesenchymal stem cells or microparticles derived from mesenchymal stem cells. The mesenchymal stem cells hardly cause a rejection even to an allogeneic subject and thus donor's cells prepared in advance, which have been expanded and cryopreserved, can be used. Likewise, microparticles derived from mesenchymal stem cells, which are donor's cells prepared in advance, which have been expanded and cryopreserved, can be used. For this reason, when compared with the case where autologous mesenchymal stem cells are prepared and used, commercialization is easier and consistent effects can be easily achieved, hence advantageous. The method of treatment can also be considered as a method for treating dilated cardiomyopathy by administering the agent for treating dilated cardiomyopathy of the present invention described above. The above description in the section of the agent for treating dilated cardiomyopathy is applicable to the mesenchymal stem cells, microparticles derived from mesenchymal stem cells and other things.

In an embodiment, not part of the invention, it is disclosed a method for suppressing fibrosis, in particular, fibrosis in dilated cardiomyopathy, comprising administering at least one selected from the group consisting of mesenchymal stem cells and microparticles derived from mesenchymal stem cells. In an embodiment, not part of the invention, it is disclosed a method for suppressing cardiac hypertrophy, in particular, cardiac hypertrophy in dilated cardiomyopathy, comprising administering at least one selected from the group consisting of mesenchymal stem cells and microparticles derived from mesenchymal stem cells. The method of suppression can also be considered as a method for suppressing fibrosis and cardiac hypertrophy by administering the agent for use in treating dilated cardiomyopathy of the present invention described above. The above description in the section of the agent for treating dilated cardiomyopathy is applicable to the mesenchymal stem cells, microparticles derived from mesenchymal stem cells and other things in the method of suppression.

The kit of the present invention may include other commercially suitable and user-friendly materials such as other additives, other agents, a diluent, a filter, a needle, a syringe and a package insert.

EXAMPLES

The present invention is specifically described with respect to the following Examples but is not limited to these Examples.

[1] Preparation of the agent for treating dilated cardiomyopathy of the present invention

(Preparation of adipose-derived mesenchymal stem cells)

After receiving consent from a human donor, subcutaneous adipose tissue obtained by a liposuction method is washed with a physiological saline solution. For achieving the destruction of extracellular matrix and isolation of the cells, collagenase (Roche) (solvent is a physiological saline solution) was added and shaken for 90 minutes at 37°C, and dispersed. Subsequently, the cells were collected and the cell suspension was centrifuged for 5 minutes at 800 g to obtain a precipitation of stromal vascular cell population. Serum-free medium for mesenchymal stem cells (Rohto Pharmaceutical Co., Ltd.) was added to the above cell precipitation, the cell suspension was centrifuged for 5 minutes at 400 g and resuspended, after removing the supernatant, in the serum-free medium for mesenchymal stem cells (Rohto Pharmaceutical Co., Ltd.), and the cells were sown in a flask. The cells were cultured at 37°C, 5% $CO_2$ for several days. Several days later, the culture was washed with PBS to remove remaining blood cells and adipose tissue contained in the culture medium and to obtain adipose-derived mesenchymal stem cells (ADSC) adhered to a plastic container.

(Cryopreservation of adipose-derived mesenchymal stem cells)

The obtained adipose-derived mesenchymal stem cells were dissociated using trypsin, moved to a centrifuge tube, and centrifuged for 5 minutes at 400 g to obtain a precipitation of cells. After removing the supernatant, a sufficient amount of a cell cryopreservation solution (STEM-CELLBANKER (ZENOAQ)) was added to suspend the cells. The cell suspension was dispensed into a cryotube, subsequently stored at -80°C in a freezer, and moved to a gas phase on liquid nitrogen to continue the storage.

(Analysis on cell surface markers (flow cytometry))

Evaluations on various surface markers on the adipose-derived mesenchymal stem cell were carried out by flow cytometry. The adipose-derived mesenchymal stem cells were resuspended in FACS staining buffer. Antibodies used for the FACS analysis were FITC (fluorescein isothiocyanate) or PE (phycoerythrin)-labelled mouse anti-human antibodies CD11b, CD45, CD73, CD90, and a corresponding mouse IgG1 isotype control antibody. Cells were stained at room temperature for 30 minutes, subsequently washed, and analyzed using BD FACSCanto II (BD Biosciences, San Jose, CA). Data were analyzed using BD FACSDiva Software (BD Biosciences). As a result, the adipose-derived mesenchymal stem cell was negative for CD45 and positive for CD73 and CD90.

(Preparation of a spray agent for treating dilated cardiomyopathy)

A fibrinogen solution and a thrombin solution for spraying were prepared using Beriplast P Combi-Set Tissue adhesion (CSL Behring Co, Ltd.). The fibrinogen solution (Beriplast solution A) contains 80 mg/mL of fibrinogen, 60IU of factor XIII, and 5,000KIE of bovine aprotinin. The thrombin solution (Beriplast solution B) contains 300 unit/mL of thrombin. Specifically, the adipose-derived mesenchymal stem cells (ADSC) which had been cryopreserved were thawed immediately before spraying, and suspended in HBSS ($\times$1) in an amount shown in Table 1 below, and Beriplast solution A was added to prepare solution A. Similarly, Beriplast solution B was added to HBSS (X1) in an amount shown in Table 1 below to prepare solution B. These solutions were contained in separate syringes and prepared for the following transplantation test.

[Table 1]

| | Beriplast solution A (Fibrinogen) | 400 $\mu$L (32 mg) |
|---|---|---|
| Solution A 2.0 mL | ADSC in HBSS (X1) (adipose-derived mesenchymal stem cell) | $1\times10^8$ cells / 1.6 mL |
| Solution B 2.0 mL | Beriplast solution B (Thrombin) | 400 $\mu$L (120 units) |
| | HBSS (X1) | 1.6 mL |

[2] Transplantation test 1

(Protocol of transplantation test)

Median sternotomy was performed for J2N-k hamsters (20 weeks old, males), and then the solutions A and B having the compositions shown in the above Table 1, which were sealed in a separate syringe, were dropped directly and simultaneously on the surface of the heart (into the pericardial membrane) at which dilated cardiomyopathy had developed to coat the area affected by diseases (ADSC group). In the group to which a medium was administered, median sternotomy was performed for J2N-k hamsters (20 weeks old, males), and then the solutions A' and B having the compositions shown in the above Table 2, which were sealed in a separate syringe, were dropped directly and simultaneously on the surface of the heart (into the pericardial membrane) at which dilated cardiomyopathy had developed to coat the area affected by diseases (control group). For sham surgery, only median sternotomy was performed for J2N-k hamsters (20 weeks old, males) and normal J2N-n hamsters (20 weeks old, males) which had not developed cardiomyopathy, and dropping of the above agent was not performed (sham group, normal, respectively).

[Table 2]

| Solution A' 2.0 mL | Beriplast solution A (fibrinogen) | 400 μL (32 mg) |
|---|---|---|
| | HBSS (X1) | 1.6 mL |
| Solution B 2.0 mL | Beriplast solution B (thrombin) | 400 μL (120 units) |
| | HBSS (X1) | 1.6 mL |

Echocardiography was carried out before transplantation (baseline), 1 week, 2 weeks, 3 weeks and 4 weeks after transplantation. At the final stage of this study, the animals were humanely sacrificed at the 4 weeks after cells transplantation for histological and biochemical analysis of the cardiac tissue. Hereinafter, the transplantation test is described in detail.

(Adipose-derived mesenchymal stem cells transplantation experiment)

Median sternotomy was performed for J2N-k hamsters (20 weeks old, males) and normal J2N-n hamsters (20 weeks old, males) under general anesthesia, and transplantation of adipose-derived mesenchymal stem cells, transplantation of medium or a sham surgery was carried out. The area at which myocardial infarction has been developed can be visually confirmed based on surface scars and abnormal wall motions. Specifically, in the ADSC group, the solutions A (20 μL) and B (20 μL) having the compositions shown in the above Table 1 (containing $1 \times 10^6$ of adipose-derived mesenchymal stem cells), which were contained in separate syringes respectively were directly sprayed at the same time on the surface of an area at which myocardial infarction has been developed (heart surface) to coat the area affected by diseases. J2N-k hamsters and J2N-n hamsters were recovered in an individual temperature-controlled cage.

(Effect of adipose-derived mesenchymal stem cells transplantation)

Effects of transplantation of adipose-derived mesenchymal stem cells on J2N-k hamsters and J2N-n hamsters were evaluated by examination of cardiac function and histopathological examination. For the examination of cardiac function, LVEF was measured by echocardiography. In the histopathological examination, cardiac hypertrophy and fibrosis were assessed. The ATP content in myocardial tissue was also measured. Furthermore, mitochondria was isolated from the heart tissue and adenine nucleotide translocator-1 (Mt. ANT-1, gene symbol: SLC25A4) expressed in mitochondria was measured by Western blot analysis. Each of the evaluation methods is described below.

(Echocardiography)

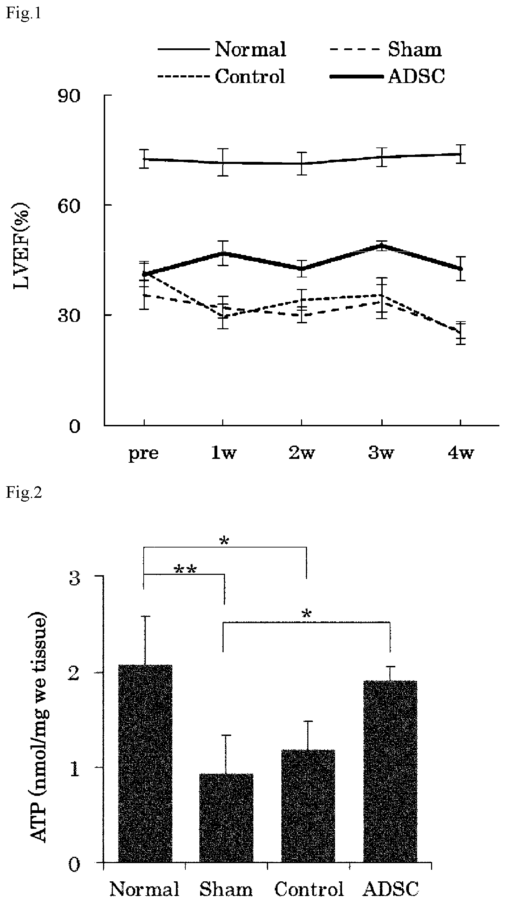

J2N-k hamsters and J2N-n hamsters were anesthetized as described above. Echocardiography was carried out using a commercial echo apparatus (HITACHI: PROSOUND F75 Premier CV). An 8.0-MHz annular array transducer was used to evaluate the heart. J2N hamsters and J2N-n hamsters were tested in the left lateral decubitus position. LV end-diastolic and end-systolic volumes (LVEDV and LVESV) were calculated by Teichholz's formula. LV ejection fraction (LVEF) was calculated from the following formula. The results are shown in Fig. 1.

$$LVEF\ (\%) = 100\ X\ (LVEDV\text{-}LVESV)\ /\ (LVEDV)$$

As shown in Fig. 1, a reduction in cardiac function was observed with reduced LVEF over time in the sham group in which only median sternotomy was performed and the control group to which a medium was administered, but LVEF was not reduced and thus the progress of dilated cardiomyopathy was suppressed in the group to which adipose-derived mesenchymal stem cells were transplanted.

(Measurement of ATP content in heart tissue)

The ATP content in the heart tissue at week 2 after the transplantation was measured by using a commercially available kit for measuring ATP. As shown in Fig. 2, the ATP content was significantly reduced in the sham group in which only median sternotomy was performed and the control group to which a medium was administered compared with normal hamsters. In contrast to this, the ATP content was not reduced and was significantly increased in the group to which adipose-derived mesenchymal stem cells were transplanted compared with the sham group.

(Measurement of Mt. ANT-1/COX4 in mitochondria in heart tissue)

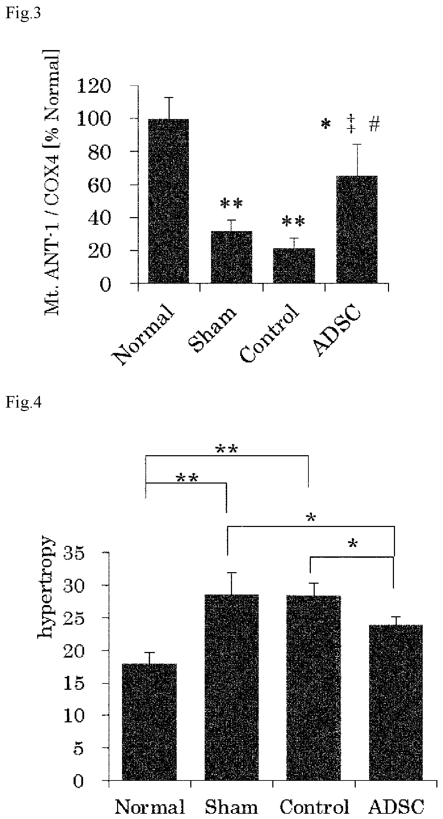

Mitochondria was isolated from the heart tissue at week 2 after the transplantation and adenine nucleotide translocator-1 (Mt. ANT-1, gene symbol: SLC25A4) expressed in mitochondria was measured by Western blot analysis. Cytochrome c oxidase 4 (COX4) was used as an endogenous control in the Western blot analysis.
As shown in Fig. 3, the expression level of Mt. ANT-1 was significantly lower in the sham group in which only median sternotomy was performed and the control group to which a medium was administered than that of normal hamsters. In contrast to this, the expression level of Mt. ANT-1 was significantly higher in the group to which adipose-derived mesenchymal stem cells were transplanted (ADSC) than that of the sham group and the control group. Mt. ANT-1 in the heart tissue at week 4 after the transplantation was measured in the same manner. The result is that similarly to the case of week 2 after the transplantation, the expression level of Mt. ANT-1 was significantly low in the sham group and the control group, but in the group to which adipose-derived mesenchymal stem cells were transplanted (ADSC), the expression level was as high as that of the normal group without any significant difference.

(Measurement of heart size)

The diameter of the heart at week 4 after the transplantation was measured using an HE stained specimen of the heart under a microscope. As shown in Fig. 4, dilated cardiomyopathy progressed and hypertrophy of the heart was observed in the sham group in which only median sternotomy was performed and the control group to which a medium was administered compared with normal hamsters. In contrast to this, hypertrophy of the heart was significantly suppressed and dilated cardiomyopathy was improved in the group to which adipose-derived mesenchymal stem cells were transplanted compared with the sham group.

(Measurement of area of fibrosis)

The area of fibrosis at week 4 after the transplantation was measured by using a Sirius red stained specimen of the heart. The area of fibrosis of the transplanted J group to which adipose-derived mesenchymal stem cells were transplanted was 78.3% based on the area of fibrosis of the sham group in which only median sternotomy was performed, revealing that adipose-derived mesenchymal stem cells suppress fibrosis in dilated cardiomyopathy.

(Measurement of expression level of δ-sarcoglycan)

Expression of δ-sarcoglycan in the heart at week 4 after the transplantation was measured by an immunostaining method. No expression of δ-sarcoglycan was observed in the sham group, the control group or the ADSC group, showing that mesenchymal stem cells have no impact on δ-sarcoglycan gene.

[3] Transplantation test 2

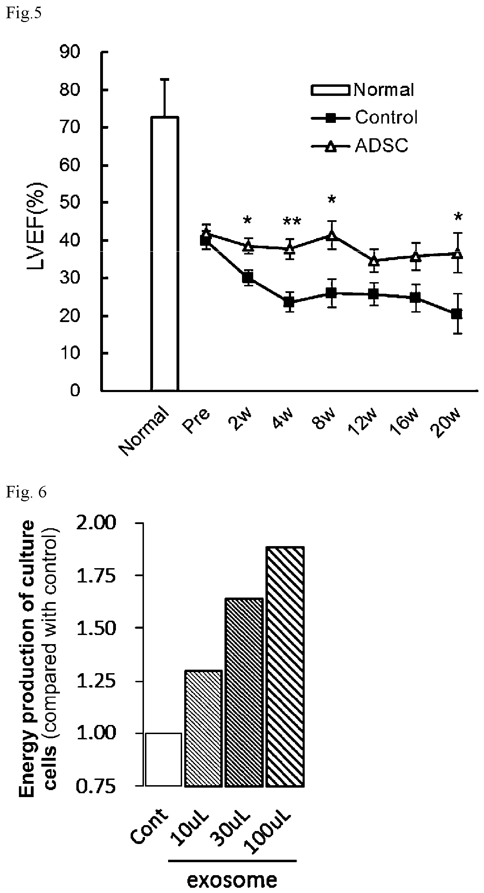

Median sternotomy was performed for J2N-k hamsters (20 weeks old, males) under general anesthesia and adipose-derived mesenchymal stem cells (ADSC) or a medium was transplanted (control) in the same manner as in Transplantation test 1. More specifically, in the same manner as in Transplantation test 1, in the ADSC group, the solution A (containing $1 \times 10^6$ adipose-derived mesenchymal stem cells) (20 μL) and solution B (20 μL) which had the composition shown in the above Table 1 and were sealed in a separate syringe, were dropped directly and simultaneously on the surface of the heart (heart surface) at which dilated cardiomyopathy had developed to coat the area affected by diseases. These J2N-k hamsters were recovered in an individual temperature-controlled cage. Echocardiography was carried out before transplantation (baseline), 2 weeks, 4 weeks, 8 weeks, 12 weeks, 16 weeks and 20 weeks after the transplantation to evaluate effects of transplantation of adipose-derived mesenchymal stem cells on J2N-k hamsters based on cardiac function. For the examination of cardiac function, LVEF was measured by echocardiography in the same manner as in Transplantation test 1. The animals used for the test were humanely sacrificed at week 20 after the cell transplantation.
As shown in Fig. 5, LVEF was reduced over time in the control group to which a medium was administered, but LVEF was not reduced and thus the progress of dilated cardiomyopathy was suppressed in the group to which adipose-derived mesenchymal stem cells were transplanted (ADSC). The effect of suppressing progress of dilated cardiomyopathy was maintained for a period of as long as 20 weeks after the transplantation.

[4] Transplantation test 3

Median sternotomy was performed for J2N-k hamsters (20 weeks old, males) under general anesthesia, and either transplantation of adipose-derived mesenchymal stem cells (ADSC) or transplantation of a medium (control) was carried out in the same manner as in Transplantation test 1. More specifically, in the same manner as in Transplantation test 1, in the ADSC group, the solution A (containing $1 \times 10^6$ adipose-derived mesenchymal stem cells) (20 $\mu$L) and solution B (20 $\mu$L) which had the composition shown in the above Table 1 and were sealed in a separate syringe, were sprayed directly and simultaneously on the surface of the heart (heart surface) at which myocardial infarction had developed to coat the area affected by diseases. The J2N-k hamsters were recovered in an individual temperature-controlled cage.

As a result, the mortality at week 12 was 28.6% in the control group and 12.5% in the ADSC administration group, showing that the mortality is reduced by transplantation of adipose-derived mesenchymal stem cells.

[5] Preparation of microparticles and study of effects on cardiomyocytes

A culture supernatant of mesenchymal stem cells prepared as described above was collected, and the culture supernatant collected was filtered through a filter (0.22 $\mu$m, Merck Millipore), and then microparticles were collected by centrifugation (35,000 rpm, 70 minutes, 4°C, BECKMAN Optima XE-90). The average particle size of the resulting microparticles was 155 $\pm$ 6 nm (average $\pm$ standard error, n = 3).

Microparticles collected from 60 mL of the culture supernatant of mesenchymal stem cells were suspended in 0.5 mL of PBS. 10 $\mu$L, 30 $\mu$L and 100 $\mu$L of the resulting microparticles derived from adipose-derived mesenchymal stem cells were each added to human-derived cardiomyocytes (available from PromoCell, $5 \times 10^5$ cells/dish). Then, NADPH activity was measured for energy production, and as a result, the energy production increased in a manner dependent on the amount of microparticles (Fig. 6). This suggests the possibility of involvement of microparticles secreted by mesenchymal stem cells in the effect of improving cardiac function of mesenchymal stem cells.

[6] Analysis of protein contained in microparticles

50 mL of Total Exosome Isolation (Thermo Fisher Scientific Inc., item number: 4478359) was added to 100 mL of a culture supernatant of mesenchymal stem cells (serum-free medium), and the mixture was thoroughly mixed by inversion. After being stored in a refrigerator overnight, the mixture was centrifuged (10,000 $\times$ g, 1 hour, 2 to 8°C), and the supernatant was removed. The sediment obtained at that stage was suspended in 500 $\mu$L of PBS to give a suspension containing microparticles. 150 $\mu$L of 20% trichloroacetic acid (TCA, available from Wako Pure Chemical Industries, Ltd.) was added to 150 $\mu$L of the suspension containing microparticles to aggregate and precipitate protein. 20 $\mu$L of tris-hydrochloric acid buffer (Tris-HCl, pH 8.5) was added to the resulting whole precipitate to dissolve the precipitate. Tris buffer containing 0.01% trypsin (available from APRO Science Inc.) (Tris-HCl, pH 8.0) was further added thereto, and reaction was carried out at 37°C for 20 minutes. Subsequently, the resulting sample solution was analyzed by LC-MS/MS (LC: available from Michrom BioResources, MS: available from Thermo Fisher Scientific), and as a result, apolipoprotein A-2, pigment epithelium-derived factor (PEDF), SERPINF 1and haptoglobin were detected.

INDUSTRIAL APPLICABILITY

According to the agent for treating dilated cardiomyopathy of the present invention, functions and the like of the heart with dilated cardiomyopathy can be notably improved. The mesenchymal stem cells hardly cause a rejection even to an allogeneic subject and thus donor's cells with confirmed treatment effects in advance is expanded, cryopreserved and can be used as the mesenchymal stem cells for the agent for treating dilated cardiomyopathy of the present invention. For this reason, when compared with the case where autologous mesenchymal stem cells are prepared and used, commercialization is easier and consistent effects can be easily achieved, hence advantageous.

**Claims**

1. An agent for use in treating dilated cardiomyopathy, comprising at least one selected from the group consisting of adipose-derived mesenchymal stem cells and microparticles derived from adipose-derived mesenchymal stem cells.

2. The agent for use in treating dilated cardiomyopathy according to claim 1, wherein the mesenchymal stem cells contain microparticles or are capable of secreting microparticles.

3. The agent for use in treating dilated cardiomyopathy according to claim 1 or 2, wherein the microparticles have an average particle size of 1,000 nm or less.

4. The agent for use in treating dilated cardiomyopathy according to any one of claims 1 to 3, wherein the microparticles are exosome.

5. The agent for use in treating dilated cardiomyopathy according to any one of claims 1 to 4, wherein the microparticles comprise at least one factor selected from the group consisting of IL-10, HGF, apolipoprotein A-2, pigment epithelium-derived factor (PEDF), SERPINF1 and haptoglobin.

6. The agent for use in treating dilated cardiomyopathy according to any one of claims 1 to 5, wherein the mesenchymal stem cells are allogeneic to a subject.

7. The agent for use in treating dilated cardiomyopathy according to any one of claims 1 to 6, wherein the dilated cardiomyopathy is a dilated cardiomyopathy selected from the group consisting of idiopathic dilated cardiomyopathy, familial dilated cardiomyopathy and inheritable dilated cardiomyopathy.

8. The agent for use in treating dilated cardiomyopathy according to any one of claims 1 to 7, wherein the mesenchymal stem cells are cryopreserved cells.

9. An agent for use in inhibiting fibrosis or cardiac hypertrophy in dilated cardiomyopathy, comprising at least one selected from the group consisting of adipose-derived mesenchymal stem cells and microparticles derived from adipose-derived mesenchymal stem cells.

10. A kit for use in treating dilated cardiomyopathy, comprising the agent for use in treating dilated cardiomyopathy according to any one of claims 1 to 8 or the agent for use in inhibiting fibrosis or cardiac hypertrophy according to claim 9, a container and a label.

**Patentansprüche**

1. Mittel zur Verwendung bei der Behandlung von dilatativer Kardiomyopathie, das zumindest eines aus der aus aus Fettgewebe stammenden mesenchymalen Stammzellen und Mikropartikeln, die von aus Fettgewebe stammenden mesenchymalen Stammzellen stammen, bestehenden Gruppe ausgewähltes umfasst.

2. Mittel zur Verwendung bei der Behandlung von dilatativer Kardiomyopathie nach Anspruch 1, wobei die mesenchymalen Stammzellen Mikropartikel enthalten oder in der Lage sind, Mikropartikel zu sezernieren.

3. Mittel zur Verwendung bei der Behandlung von dilatativer Kardiomyopathie nach Anspruch 1 oder 2, wobei die Mikropartikel eine durchschnittliche Teilchengröße von 1.000 nm oder weniger aufweisen.

4. Mittel zur Verwendung bei der Behandlung von dilatativer Kardiomyopathie nach einem der Ansprüche 1 bis 3, wobei die Mikropartikel Exosomen sind.

5. Mittel zur Verwendung bei der Behandlung von dilatativer Kardiomyopathie nach einem der Ansprüche 1 bis 4, wobei die Mikropartikel zumindest einen Faktor umfassen, der aus der aus IL-10, HGF, Apolipoprotein A-2, dem vom Pigmentepithel abgeleiteten Faktor (PEDF), SERPINF1 und Haptoglobin bestehenden Gruppe ausgewählt ist.

6. Mittel zur Verwendung bei der Behandlung von dilatativer Kardiomyopathie nach einem der Ansprüche 1 bis 5, wobei die mesenchymalen Stammzellen allogen für ein Individuum sind.

7. Mittel zur Verwendung bei der Behandlung von dilatativer Kardiomyopathie nach einem der Ansprüche 1 bis 6, wobei die dilatative Kardiomyopathie eine dilatative Kardiomyopathie ist, die aus der aus idiopathischer dilatativer Kardiomyopathie, familiärer dilatativer Kardiomyopathie und vererbbarer dilatativer Kardiomyopathie bestehenden Gruppe ausgewählt ist.

8. Mittel zur Verwendung bei der Behandlung von dilatativer Kardiomyopathie nach einem der Ansprüche 1 bis 7, wobei die mesenchymalen Stammzellen kryokonservierte Zellen sind.

9. Mittel zur Verwendung bei der Hemmung von Fibrose oder kardialer Hypertrophie bei dilatativer Kardiomyopathie, das zumindest eines aus der aus aus Fettgewebe stammenden mesenchymalen Stammzellen und Mikropartikeln, die von aus Fettgewebe stammenden mesenchymalen Stammzellen stammen, bestehenden Gruppe ausgewähltes umfasst.

10. Set zur Verwendung bei der Behandlung von dilatativer Kardiomyopathie, welches ein Mittel zur Verwendung bei der Behandlung von dilatativer Kardiomyopathie nach einem der Ansprüche 1 bis 8 oder ein Mittel zur Verwendung bei der Hemmung von Fibrose oder kardialer Hypertrophie nach Anspruch 9, einen Behälter und ein Etikett umfasst.

**Revendications**

1. Agent pour utilisation dans le traitement de la cardiomyopathie dilatée, comprenant au moins un choisi dans le groupe constitué de cellules souches mésenchymateuses dérivées d'adipose et de microparticules dérivées de cellules souches mésenchymateuses dérivées d'adipose.

2. Agent pour utilisation dans le traitement d'une cardiomyopathie dilatée selon la revendication 1, dans lequel les cellules souches mésenchymateuses contiennent des microparticules ou sont capables de sécréter des microparticules.

3. Agent pour utilisation dans le traitement de la cardiomyopathie dilatée selon la revendication 1 ou 2, dans lequel les microparticules présentent une taille de particule moyenne de 1 000 nm ou moins.

4. Agent pour utilisation dans le traitement de la cardiomyopathie dilatée selon l'une quelconque des revendications 1 à 3, dans lequel les microparticules sont des exosomes.

5. Agent pour utilisation dans le traitement de la cardiomyopathie dilatée selon l'une quelconque des revendications 1 à 4, dans lequel les microparticules comprennent au moins un facteur choisi dans le groupe constitué de IL-10, HGF, apolipoprotéine A-2, facteur dérivé de l'épithélium pigmentaire (PEDF), SERPINF1 et haptoglobine.

6. Agent pour utilisation dans le traitement de la cardiomyopathie dilatée selon l'une quelconque des revendications 1 à 5, dans lequel les cellules souches mésenchymateuses sont allogéniques par rapport à un sujet.

7. Agent pour utilisation dans le traitement de la cardiomyopathie dilatée selon l'une quelconque des revendications 1 à 6, dans lequel la cardiomyopathie dilatée est une cardiomyopathie dilatée choisie dans le groupe constitué de la cardiomyopathie dilatée idiopathique, de la cardiomyopathie dilatée familiale et de la cardiomyopathie dilatée héréditaire.

8. Agent pour utilisation dans le traitement de la cardiomyopathie dilatée selon l'une quelconque des revendications 1 à 7, dans lequel les cellules souches mésenchymateuses sont des cellules cryoconservées.

9. Agent pour utilisation dans l'inhibition de la fibrose ou de l'hypertrophie cardiaque dans la cardiomyopathie dilatée, comprenant au moins certaines choisies dans le groupe constitué de cellules souches mésenchymateuses dérivées d'adipose et de microparticules dérivées de cellules souches mésenchymateuses dérivées d'adipose.

10. Kit pour utilisation dans le traitement de la cardiomyopathie dilatée, comprenant l'agent pour utilisation dans le traitement de la cardiomyopathie dilatée selon l'une quelconque des revendications 1 à 8 ou l'agent pour utilisation dans l'inhibition de la fibrose ou de l'hypertrophie cardiaque selon la revendication 9, un récipient et un marqueur.

Fig.1

Fig.2

Fig.3

Fig.4

Fig.5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002506831 A
- JP 2000508911 A
- JP 2012157263 A
- JP 2012508733 A
- US 20150125950 A1
- US 20110003008 A1
- WO 2015076717 A2
- WO 2014013029 A1
- US 6777231 B
- WO 2007058308 A

### Non-patent literature cited in the description

- **HITONOBU TOMOIKE**. *Guidelines for Diagnosis and Treatment of Cardiovascular Diseases*, 2011
- *Mol. Cell Biochem.*, 2014, vol. 387, 279-285
- *Med. Sci. Monit. Basic Res.*, 2013, vol. 14, 20-31
- *Circ. J.*, 2005, vol. 69, 107-113
- *J. Biochem.*, 2003, vol. 34 (2), 269-76
- *JCI.*, 2000, vol. 106, 655-662
- **PITTENGER F. M. et al.** *Science*, 1999, vol. 284, 143-147
- *Circ.*, 2005, vol. 112, 1128-1135
- *Mesenchymal Stem Cells for Idiopathic Dilated Cardiomyopathy*, 08 October 2023